# EUROPÄISCHE PATENTSCHRIFT

(11) **EP 3 634 368 B1**
(45) Veröffentlichungstag und Bekanntmachung des Hinweises auf die Patenterteilung: **17.03.2021**
(21) Anmeldenummer: 17731831.8
(22) Anmeldetag: 07.06.2017
(51) Int. Cl.: C11D 3/50, A61K 8/36, A61K 8/42, A61Q 13/00, A61Q 19/00, A61K 8/02, A61K 8/11, A61K 8/19, A61K 9/16, A61Q 5/00

(54) **PULVER ENTHALTEND KRISTALLE MIT DARIN EINGESCHLOSSENEN INHALTSSTOFFEN**
POWDER CONTAINING CRYSTALS COMPRISING INGREDIENTS ENCLOSED THEREIN
POUDRE CONTENANT DES CRISTAUX ET DES INGRÉDIENTS INCORPORÉS DANS CELLE-CI

(43) Veröffentlichungstag der Anmeldung: 15.04.2020
(73) Patentinhaber: Symrise AG, 37603 Holzminden (DE)
(72) Erfinder: BEHRENS, Kolja, 37647 Polle (DE); OTT, Patrick, 37603 Holzminden (DE); PICHON, Nicolas, 37671 Lüchtringen (DE); SCHMIDTMEIER, Nadine, 32676 Lügde (DE); WIEDEMANN, Jörn, 37603 Holzminden (DE)
(74) Vertreter: Eisenführ Speiser
(86) Internationale Anmeldenummer: PCT/EP2017/063839
(87) Internationale Veröffentlichungsnummer: WO 2018/224142

(56) Entgegenhaltungen:
- WO-A1-2008/065563
- WO-A1-2014/085286
- WO-A2-2014/047496

## Beschreibung

Die vorliegende Erfindung betrifft primär ein Verfahren zur Herstellung eines Pulvers bestehend aus oder umfassend eine Vielzahl von in Kristallen eingeschlossenen, einen oder mehrere Inhaltsstoffe enthaltenden Kapseln.

Die Erfindung betrifft zudem ein solches Pulver, bestehend aus oder umfassend eine Vielzahl von in Kristallen eingeschlossenen, einen oder mehrere Inhaltsstoffe enthaltenden Kapseln, darüber hinaus Produkte umfassend ein solches Pulver, die Verwendung eines solchen Pulvers, vorzugsweise in solchen Produkten sowie Verfahren zum Herstellen eines solchen Produktes und Verfahren zum Parfümieren von Substraten.

Weitere Aspekte und bevorzugte Ausgestaltungen der vorliegenden Erfindung ergeben sich aus den nachfolgenden Ausführungen, den beigefügten Beispielen und insbesondere den beigefügten Patentansprüchen.

Das Einarbeiten von Kapseln, z.B. Duftöl enthaltenden Kapseln, in Waschpulver kann beispielsweise durch das Aufsprühen von Kapseln auf das fertige Waschpulver erfolgen. Hierbei sind die Kapseln jedoch ungeschützt und die Wahrscheinlichkeit ist sehr hoch, dass ein Teil der Kapseln während des weiteren Herstellungsprozesses (z.B. Mischen und Fördern des Pulvers) durch auftretende Scherkräfte in dem Waschpulver zerrieben wird. Sofern die Kapseln nicht ausreichend an den Waschpulverpartikeln anhaften, kann es außerdem zu einer Separation der Kapseln im Pulver kommen. Dadurch ist die Verteilung der Kapseln im Waschpulver nicht mehr gleichmäßig. Eine weitere Methode, um Kapseln in Waschpulver einzuarbeiten, besteht darin, dass Kapseln zusammen mit anderen Komponenten des Waschpulvers zusammen sprühgetrocknet werden. Hierbei werden die Kapseln jedoch erheblich thermisch belastet. Durch diese thermische Belastung besteht das Risiko, die Qualität der eingesetzten Kapseln zu verändern oder sie sogar zu zerstören.

Die primäre Aufgabe der vorliegenden Erfindung lag nun darin, ein Verfahren zur Herstellung eines Pulvers bereitzustellen, bei dem die o.g. Nachteile nicht oder nur in sehr reduziertem Maße auftreten. Weitere Aspekte dieser Aufgabe sowie weitere Aufgabenstellungen ergeben sich aus den nachfolgenden Ausführungen.

Erfindungsgemäß gelöst wird die primäre gestellte Aufgabe durch ein Verfahren zur Herstellung eines Pulvers bestehend aus oder umfassend eine Vielzahl von in Kristallen eingeschlossenen, einen oder mehrere Inhaltsstoffe enthaltenden Kapseln (K), bestehend aus oder umfassend folgende Schritte:
(i) Bereitstellen von einem oder mehreren Lösemitteln,
   Bereitstellen von einem oder mehreren kristallisierbaren Materialien und
   Bereitstellen von einem oder mehreren einen oder mehrere Inhaltsstoffe enthaltenden Kapseln (K),
(ii) optional: Temperaturerhöhung des/der Lösemittel(s),
(iii) Hinzufügen
   (iii.1) des/der bereitgestellten kristallisierbaren Materials/Materialien und
   (iii.2) optional: der bereitgestellten Kapseln (K), vor, nach oder gleichzeitig mit dem/den kristallisierbaren Materialien,
   zu dem/den Lösemittel(n) und Lösen des/der bereitgestellten kristallisierbaren Materials/Materialien in dem/den Lösemittel(n), um eine Mischung (M1) zu erhalten,
(iv) Reduzieren der Temperatur der Mischung (M1) auf eine Temperatur unterhalb der oder bis zur Kristallisation des/der bereitgestellten kristallisierbaren Materials/Materialien,
(v) Hinzufügen der bereitgestellten Kapseln (K) bzw. weiterer Kapseln (K), wobei Schritt (v) im Falle der Durchführung des Schrittes (iii.2) optional ist,
(vi) weiteres Reduzieren der Temperatur der Mischung (M1), um eine Mischung (M2) zu erhalten, die eine Vielzahl von in Kristallen eingeschlossenen, einen oder mehrere Inhaltsstoffe enthaltenden Kapseln (K), umfasst,
(vii) Sammeln von in Kristallen eingeschlossenen, einen oder mehrere Inhaltsstoffe enthaltenden Kapseln (K).

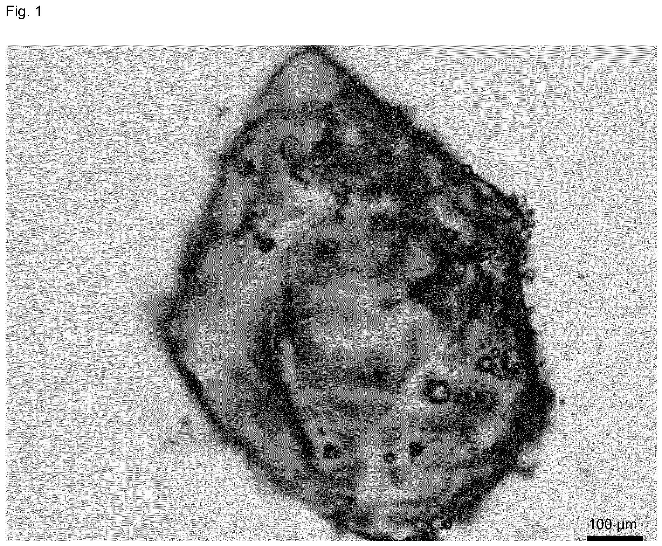

Die Kapseln (K) werden während des Kristallisationsvorgangs zumindest teilweise in und/oder von den sich bildenden Kristallen eingeschlossen (vgl. Fig. 1).

Vorzugsweise liegt/liegen das/die kristallisierbare(n) Material(ien) in einer Konzentration in dem/den Lösemittel(n) vor, die die Kristallisation bei einer Temperatur einsetzen lässt, die über dem Gefrierpunkt des/der Lösemittel(s) liegt.

Die in Schritt (i) bereitgestellten, einen oder mehrere Inhaltsstoffe enthaltenden Kapseln (K) können als Pulver, in einer Dispersion oder in anderer Form vorliegen.

Unter dem Einsetzen der Kristallisation versteht man den Zeitpunkt, an dem erste Kristalle bei Betrachten der Mischung M1 mit bloßem Auge sichtbar werden.

Vorzugsweise wird in einem erfindungsgemäßen Verfahren Schritt (v) unmittelbar nach Schritt (iv) durchgeführt.

Die erfindungsgemäß hergestellten Kristalle bzw. ein erfindungsgemäß hergestelltes Pulver lassen sich vorteilhafterweise problemlos mit fertigen Waschpulverformulierungen bzw. üblichen Bestandteilen eines Waschpulvers mischen. Hierbei sind die enthaltenen bzw. eingeschlossenen Kapseln beim Einarbeiten in das Waschpulver besser vor mechanischer Belastung geschützt als Kapseln, die lose in das Waschpulver eingearbeitet werden.

Bei Bedarf kann die Partikelgröße der erfindungsgemäß hergestellten Kristalle durch Einstellung der entsprechenden Parameter (z.B. Temperatur bzw. Temperarturprofil während des Prozesses und die Prozessdauer) an die Korngröße des Waschpulvers angepasst werden, so dass Separationsphänomene in der Waschpulverzubereitung vermieden werden.

Außerdem werden starke thermische Belastungen, wie sie z.B. beim Sprühtrocknen auftreten, vermieden.

Aufgrund der Tatsache, dass das erfindungsgemäß hergestellte Produkt ein Pulver ist, erleichtert es im Vergleich zu beispielsweise flüssigen Kapseldispersionen den notwendigen Aufwand bei Transport und Verpackung erheblich.

Die in Schritt (i) bereitgestellten und einen oder mehrere Inhaltsstoffe enthaltenden Kapseln (K) können beispielsweise einen oder mehrere Geruchsstoffe enthalten:
Erfindungsgemäß bevorzugt eingesetzte Geruchsstoffe umfassen beispielsweise Parfümöle, die beispielsweise aus folgenden Rohstoffen zusammengesetzt sind bzw. werden:
- Extrakte aus natürlichen Rohstoffen wie Etherische Öle, Concretes, Absolues, Resine, Resinoide, Balsame, Tinkturen wie z.B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-ÖI; Eucalyptusöl; Fenchelöl; Fichtennadelöl; Galbanumöl; Galbanumresin; Geraniumöl; Grapefruitöl; Guajakholzöl; Gurjunbalsam; Gurjunalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierte Inhaltsstoffe;
- Einzel-Riechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
- aliphatische Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
- aliphatische Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
- aliphatische schwefelhaltige Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
- aliphatische Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
- Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat, insbesondere Ethyl-2-trans-4-cis-decadienoat; Methyl-2-octinat; Methyl-2-noninat; Allyl-2-isoamyloxyacetat; Methyl-3,7-dimethyl-2,6-octadienoat; 4-Methyl-2-pentyl-crotonat;
- Formiate, Acetate, Propionate, Isobutyrate, Butyrate, Isovalerianate, Pentanoate, Hexanoate, Crotonate, Tiglinate oder 3-Methyl-2-butenoate von acyclischen Terpenalkoholen wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol;
- acyclische Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton sowie deren Dimethyl- und Diethylacetale; insbesondere die Dimethyl- und Diethylacetale von Geranial, Neral, 7-Hydroxy-3,7-dimethyloctanal;
- Formiate, Acetate, Propionate, Isobutyrate, Butyrate, Isovalerianate, Pentanoate, Hexanoate, Crotonate, Tiglinate und 3-Methyl-2-butenoate von cyclischen Terpenalkoholen wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol;
- cyclische Terpenaldehyde und -ketone wie z.B. Menthon; Isomenthon; 8-Mercaptomenthan-3-on; Carvon; Campher; Fenchon; alpha-lonon; beta-lonon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
- cyclische und cycloaliphatische Ether wie z.B. Cineol; Cedrylmethylether; Cyclododecylmethylether;1,1-Dimethoxycyclododecan; (Ethoxy-methoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
- cyclische und makrocyclische Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cycloheptadecen-1-on; Cyclopentadecanon; Cyclohexadecanon;
- cycloaliphatische Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
- cycloaliphatische Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
- Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahyd ro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methano-octahydro-5, bzw. 6-indenylacetat;
- Ester cycloaliphatischer Alkohole wie z.B.1-Cyclohexylethylcrotonat;
- Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
- Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
- araliphatische Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
- aromatische und araliphatische Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl) propanal;
- aromatische und araliphatische Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl] ethanon; 5',6',7',8'-Tetrahydro-3' ,5' ,5' ,6' ,8' ,8'-hexamethyl-2-acetonaphthon;
- aromatische und araliphatische Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
- stickstoffhaltige aromatische Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
- Phenylether und Phenylester wie z.B. Estragol; Anethol; Eugenylmethylether; Isoeugenylmethylether; Diphenylether; beta-Naphthylmethylether; beta-Naphthylethylether; beta-Naphthylisobutylether; 1,4-Dimethoxybenzol; Eugenylacetat; p-Kresylphenylacetat;
- heterocyclische Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on; und
- Dihydrocumarin; Octahydrocumarin; Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.
   Ebenfalls als Duftstoff geeignet sind sogenannte Duftstoffvorläufer (Pro-Drug). Bei dieser Klasse von Verbindungen handelt es sich um Verbindungen, welche durch das Aufbrechen einer chemischen Bindung, beispielsweise durch Hydrolyse, ein erwünschtes Geruchs- und/oder Duftstoffmolekül freisetzt. Typischerweise wird zur Bildung eines Duftstoffvorläufers ein gewünschtes Duftstoffrohmaterial chemisch mit einem Träger, vorzugsweise einem geringfügig flüchtigen oder mäßig flüchtigen Träger, verbunden. Die Kombination führt zu einem weniger flüchtigen und stärker hydrophoben Duftstoffvorläufer mit verbesserter Anlagerung auf Stoffen. Der Duftstoff wird danach durch Aufbrechen der Bindung zwischen dem Duftstoffrohmaterial und dem Träger freigesetzt, beispielsweise durch eine Veränderung des pH-Werts (z. B. durch Transpiration beim Tragen), Luftfeuchtigkeit, Wärme und/oder Sonnenlicht während der Lagerung oder des Trocknens auf der Wäscheleine.

Zudem oder alternativ können ein oder mehrere Insektenrepellentien enthalten sein, z.B. N,N-Diethyl-m-toluamid, 1,2-Pentandiol und/oder Ethyl Butylacetylaminopropionate.

Zudem oder alternativ können ein oder mehrere Kühlstoffe enthalten sein. Kühlstoffe sind Verbindungen, die auf der Haut ein Gefühl der Kälte erzeugen. In der Regel handelt es sich dabei um Mentholverbindungen, die - neben dem Grundkörper Menthol selber - beispielsweise ausgewählt sind aus der Gruppe, die gebildet wird von Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und - amiden WS-3, WS-4, WS-5, WS-12, WS-14 und WS-30 sowie deren Gemischen. Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:
Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonate erwiesen, die die Anmelderin unter den Bezeichnungen Frescolat® MGA, Frescolat® ML, Frecolat® MGC und Frescolat® MPC vertreibt. In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

Zudem oder alternativ können ein oder mehrere Stoffe genutzt werden, die der Fachmann als TRPV1 und TRPV3 Modulatoren bezeichnet. Beispiele für solche Substanzen sind: Vanilly derivate, vorzugsweise Vanillylether, Capsaicin, Allyl isothiocyanate, Gingerol, 4-(I-menthoxymethyl)-2-phenyl-1,3-dioxolan,4-(I-menthoxymethyl)-2-(3',4'-dihydroxyphenyl)-I,3-dioxolan, 4-(I-menthoxymethyl)-2- (2'-hydroxy-3 '-methoxyphenyl)-3-dioxolan, 4-(I-menthoxymethyl)-2-(4'-methoxyphenyl)-3-dioxolan, 4-(I-menthoxymethyl)-2-(3',4'me-thylene-dioxyphenyl)-3 -dioxolan, 4-(I-menthoxymethyl)-2-(3 '-methoxy-4'-hydroxyphenyl) -3 -dioxolan, Red pepper oil, Red pepper oleoresin, Ginger oleoresin, nonylic acid vanillyl amide, Jambu oleoresin, Zanthoxylum piperitum extract, Sanshool I, Sanshool II, Sanshoamide, Black pepper extract, Chavicine und weitere Substanzen, wie sie beispielsweise in der Patentschrift US 6780443 erwähnt werden.

Zudem oder alternativ können ein oder mehrere Geschmacksstoffe, ein oder mehrere kosmetische Inhaltsstoffe, ein oder mehrere pharmazeutische Wirkstoffe, ein oder mehrere Latentwärmespeicher, ein oder mehrere Adsorbentien und/oder ein oder mehrere Inhaltsstoffe für Mund- und Zahnpflegemittel enthalten sein.

Im Ergebnis enthalten in einem erfindungsgemäßen Verfahren, vorzugsweise in einer vorstehend als bevorzugt bezeichneten Ausführungsform, vorzugsweise mehrere oder sämtliche der Kapseln (K) einen oder mehrere Inhaltsstoffe aus der Gruppe bestehend aus Geruchsstoffen, Geschmacksstoffen, kosmetischen Inhaltsstoffen, pharmazeutischen Wirkstoffen, Insektenrepellentien, Inhaltsstoffen für Mund- und Zahnpflegemittel, Latentwärmespeichern und Adsorbentien.

In einer bevorzugten Ausgestaltung des erfindungsgemäßen Verfahrens, vorzugsweise in einer vorstehend als bevorzugt bezeichneten Ausführungsform, weisen mehrere oder sämtliche der Kapseln (K) ein Wandmaterial auf, das aus einem oder mehreren der Substanzen ausgewählt aus der Gruppe bestehend aus natürlichen, halbsynthetischen und vollsynthetischen Hüllmaterialien, bevorzugt aus Melamin-Formaldehyd Harzen, Gelatine, Alginaten, Polyurethanen, Polyamiden und Polyharnstoffen besteht oder eine oder mehrere solche Substanzen umfasst oder auf einer oder mehreren solchen Substanzen basiert.

Die in Schritt (i) bereitgestellten Kapseln verfügen vorzugsweise über eine Kapselwand aus für den gewünschten Einsatzzweck geeignetem Material. Als Kapselwandmaterial geeignet können z.B. natürliche Hüllmaterialien wie beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse, halbsynthetische Hüllmaterialien wie unter anderem chemisch modifizierte Cellulosen, insbesondere Celluloseester und ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester, oder vollsynthetische Hüllmaterialien, beispielsweise Polymere wie Polyacrylate, Polyamide, Polyvinylalkohol, Aminoplaste, Phenoplaste oder Polyvinylpyrrolidon, sein.

Kapseln (K), die für die Zwecke der vorliegenden Erfindung eingesetzt werden können, sind im Handel erhältlich, z.B. folgende Handelsprodukte (in Klammern angegeben ist jeweils das Hüllmaterial): Hallcrest Microcapsules (Gelatine, Gum-mi Arabicum), Coletica Thalaspheres (maritimes Collagen), Lipotec Millicapseln (Alginsäure, Agar-Agar), Induchem Unispheres (Lactose, mikrokristalline Cellulose, Hydroxypropylmethylcellulose); Unicerin C30 (Lactose, mikrokristalline Cellulose, Hydroxypropylmethylcellulose), Kobo Glycospheres (modifizierte Stärke, Fettsäureester, Phospholipide), Softspheres (modi-fiziertes Agar-Agar) und Kuhs Probiol Nanospheres (Phospholipide) sowie Primaspheres und Primasponges (Chitosan, Alginate) und Primasys (Phospholipide). Sowie Kapseln aus synthethischen Polymeren Micronal® (BASF), Mikrokapseln 500 und 560 (Koehler SE), Folco Smart-caps®, Enfinit™, Ensensa. Alternativ können die Kapseln (K) auch, vorzugsweise unter Berücksichtigung der oben beschriebenen bevorzugten Hüllmaterialien und Inhaltsstoffe, selbst hergestellt werden. Dem Fachmann sind geeignete Methoden hierfür bekannt, z.B. durch Grenzflächenpolymerisation (z.B. J. Li, A. P. Hitchcock, H.D.H Stöver, I. Shirley; Macromolecules; 2009;42;2428-2432) oder in-situ Polymerisation (wie z.B. DE19835114A1).

Gemäß einer bevorzugten Ausgestaltung der vorliegenden Erfindung werden die Kapseln (K) in Form einer Kapseldispersion zur weiteren Verwendung in einem erfindungsgemäßen Verfahren (wie hierin beschrieben) bereitgestellt. Eine solche kann beispielsweise hergestellt werden, indem eine aus einer Lösung kristallisierbare Substanz in einem geeigneten Lösungsmittel gelöst wird. Anschließend werden die Kapseln zugesetzt und es werden Bedingungen geschaffen, unter denen die gelöste Substanz wieder auskristallisiert.

In einer bevorzugten Ausgestaltung des erfindungsgemäßen Verfahrens ist/sind das/ein, mehrere oder sämtliche Lösemittel ausgewählt aus der Gruppe bestehend aus Wasser und mit Wasser mischbaren Lösungsmitteln, bevorzugt Ethanol, Isopropanol, Aceton und DMSO.

Besonders vorteilhaft sind solche Lösemittel auszuwählen, in denen das/die kristallisierbaren Material(ien) besonders gut kristallisiert/kristallisieren.

In einem erfindungsgemäßen Verfahren, insbesondere in einer bevorzugten Ausgestaltung, lässt/lassen sich das/ein, mehrere oder sämtliche kristallisierbare Material(ien) in einem mit Wasser mischbaren Lösungsmittel oder mit Wasser kristallisieren und/oder ist/sind bevorzugt ausgewählt aus der Gruppe bestehend aus Salzen, bevorzugt Alkali- und Erdalkalimetallsalze, besonders bevorzugt Natriumsalze, insbesondere bevorzugt Natriumsulfat, Natriumchlorid oder Natriumcarbonat, Zuckern und Harnstoff.

Besonders vorteilhaft sind lösliche Salze, Zucker oder Harnstoff, da sie sich bei erfindungsgemäßer Verwendung des Pulvers in beispielsweise Wasser oder mit Wasser mischbaren Flüssigkeiten gut auflösen und die beinhalteten Kapseln (K) leicht freigeben.

In einem erfindungsgemäßen Verfahren, insbesondere in einer vorstehend als bevorzugt bezeichneten Ausgestaltung, wird/werden in Schritt (ii), sofern vorhanden, das/die Lösemittel auf eine Temperatur im Bereich von 23 bis 50 °C, vorzugsweise im Bereich von 25 bis 40 °C, besonders bevorzugt im Bereich von 30 bis 35 °C, erhöht bzw. erhitzt.

Wird/werden das/die Lösemittel auf eine solche Temperatur erhöht bzw. erhitzt, so lösen sich das/die kristallisierbare(n) Material(ien) vorteilhafterweise besonders gut bzw. besonders schnell in dem/den Lösemittel(n).

Bevorzugt umfasst in einem erfindungsgemäßen Verfahren, vorzugsweise in einer vorstehend als bevorzugt bezeichneten Ausführungsform, Schritt (iii) ein Rühren, um das/die kristallisierbare(n) Material(ien) in dem/den Lösemitteln zu lösen oder dies zu erleichtern, vorzugsweise mit einer Rührgeschwindigkeit im Bereich von 100 bis 1.871 U/Min., bevorzugt im Bereich von 500 bis 1300 U/Min., besonders bevorzugt im Bereich von 1000 bis 1200 U/Min., vorzugsweise mittels eines Leitstrahlrührers.

In einer bevorzugten Ausgestaltung des erfindungsgemäßen Verfahrens, vorzugsweise in einer vorstehend als bevorzugt bezeichneten Ausführungsform, wird in Schritt (vi) die Temperatur der Mischung (M1) auf eine Temperatur von < 20 °C, vorzugsweise < 15 °C, weiter vorzugsweise < 10 °C, besonders bevorzugt < 5 °C, reduziert, wobei das Reduzieren der Temperatur vorzugsweise über einen Zeitraum im Bereich von 0,25 bis 3 Stunden, bevorzugt 0,5 bis 2 Stunden, besonders bevorzugt über einen Zeitraum von 0,75 bis 1,5 Stunden, erfolgt.

Vorteilhafterweise findet die Kristallisation des/der kristallisierbaren Materials/Materialien bei den oben genannten Temperaturen und über den oben genannten Zeitraum, statt. Bei einem Reduzieren der Temperatur über einen Zeitraum in den oben beschriebenen Bereichen läuft die Kristallisation des/der kristallisierbaren Materials/Materialien unter besonders vorteilhaften Bedingungen ab und führt zu der Bildung von Strukturen, die den Einschluss der einen oder mehrere Inhaltsstoffe enthaltenden Kapseln (K) in die entstehenden Kristalle besonders gut gewährleistet, was bei erfindungsgemäßer Verwendung wiederum zu einem Schutz der Kapseln (K) vor beispielsweise Zerreiben durch auftretende Scherkräfte führt.

Bevorzugt umfasst oder besteht Schritt (vii) des erfindungsgemäßen Verfahrens, vorzugsweise in einer vorstehend als bevorzugt bezeichneten Ausführungsform, aus einem, zwei oder sämtlichen der folgenden Schritte:
- Dekantieren des/der Lösemittel(s),
- Abtrennen (z.B. durch Abfiltrieren und oder Zentrifugieren) der Kristalle,
- Trocknen der in Schritt (vi) erhaltenen Mischung (M2) und/oder der abfiltrierten Kristalle,
um ein Pulver bestehend aus oder umfassend eine Vielzahl von in Kristallen eingeschlossenen, einen oder mehrere Inhaltsstoffe enthaltenden Kapseln (K) zu erhalten.

Durch das bevorzugte Dekantieren und/oder Abfiltrieren und/oder Trocknen in Schritt (vii) wird ein erfindungsgemäßes Pulver erhalten, welches sich aufgrund seiner Pulverform besonders vorteilhaft zu verschiedenen flüssigen oder festen Produkten hinzufügen bzw. mit diesen mischen lässt.

In einem erfindungsgemäßen Verfahren, insbesondere in einer bevorzugten Ausgestaltung, liegt in Schritt (i) das Gewichtsverhältnis der Gesamtmenge an Lösemittel(n) zur Gesamtmenge an kristallisierbarem/n Material(ien) zur Gesamtmenge an Kapseln (K) und/oder Kapseldispersion (vgl. oben), in folgendem Bereich: 1-10 : 0.5-5 : 0.1-3, vorzugsweise in folgendem Bereich: 3-7 : 1-3 : 0.5-2.

Die oben genannten Bereiche der Gewichtsverhältnisse sind besonders vorteilhaft, da in diesen Bereichen eine für den Kristallisationsprozess besonders vorteilhafte Menge an kristallisierbarem/n Material(ien) in dem/den Lösemittel(n) vorliegt und die entstehenden Kristalle eine besonders vorteilhafte Menge an Kapseln (K) einschließen können, so dass eine für die erfindungsgemäße Verwendung besonders vorteilhafte Menge an Kapseln (K) im entstandenen Pulver vorhanden ist, bei der Herstellung dessen aber kein unnötiger Verlust der eingesetzten Kapseln (K) entsteht.

Ein weiterer Aspekt der vorliegenden Erfindung betrifft ein Pulver bestehend aus oder umfassend eine Vielzahl von in Kristallen eingeschlossenen, einen oder mehrere Inhaltsstoffe enthaltenden Kapseln, vorzugsweise hergestellt oder herstellbar durch ein erfindungsgemäßes Verfahren, vorzugsweise in einer bevorzugten Ausgestaltung.

Für bevorzugte Ausgestaltungen eines solchen Pulvers gilt das oben im Zusammenhang mit einem erfindungsgemäßen Verfahren Gesagte entsprechend, insbesondere das im Zusammenhang mit bevorzugten Ausgestaltungen Gesagte, ganz besonders betreffend die erfindungsgemäß (bevorzugt) zu verwendenden Kapseln, deren Hüllmaterialien und Inhaltsstoffe sowie die (bevorzugt) zu verwendenden kristallisierbaren Materialien.

Wie oben erwähnt, lassen sich die erfindungsgemäß hergestellten Kristalle bzw. ein erfindungsgemäß hergestelltes Pulver vorteilhafterweise problemlos mit einer fertigen Waschpulverformulierung bzw. üblichen Bestandteilen eines Waschpulvers mischen. Doch auch andere Produkte können die hierin beschriebenen Kristalle bzw. Pulver enthalten.

Demnach betrifft ein weiterer Aspekt der vorliegenden Erfindung ein Produkt ausgewählt aus der Gruppe bestehend aus Wasch- und Reinigungsmitteln, Körperpflegeprodukten, der Ernährung oder dem Genuss dienenden Zubereitungen, kosmetischen oder pharmazeutischen Zubereitungen, parfümierten, parfümierenden oder zu parfümierenden Produkten, vorzugsweise Duftspülern, wobei das Produkt ein erfindungsgemäßes Pulver umfasst.

Neben einem hierin beschriebenen Pulver enthalten solche Produkte im Übrigen die für solche Produkte üblichen und geeigneten weiteren Inhaltsstoffe. Diese sind dem Fachmann auf dem jeweiligen Gebiet bestens bekannt und werden je nach gewünschter Anwendung entsprechend ausgewählt.

Erfindungsgemäße Produkte enthalten daher vorzugsweise zudem einen oder mehrere übliche Bestandteile bzw. Inhaltsstoffe von Wasch- und Reinigungsmitteln, Körperpflegeprodukten, der Ernährung oder dem Genuss dienenden Zubereitungen, kosmetischen oder pharmazeutischen Zubereitungen oder parfümierten, parfümierenden oder zu parfümierenden Produkten.

Weiterhin betrifft ein Aspekt der vorliegenden Erfindung die Verwendung eines erfindungsgemäßen Pulvers, wobei mehrere oder sämtliche der Kapseln (K) einen oder mehrere Inhaltsstoffe aus der Gruppe bestehend aus Kühlwirkstoffen, Geruchsstoffen (vorzugsweise wie oben beschrieben) und Geschmacksstoffen enthalten, vorzugsweise in einer Menge, die ausreicht, um eine Kühlwirkung hervorzurufen und/oder um einen sensorischen, vorzugsweise einen Geruchs- und/oder Geschmackseindruck zu vermitteln, zu modifizieren oder zu verstärken, zum Vermitteln, Modifizieren oder Verstärken eines sensorischen, vorzugsweise eines Geruchs- und/oder Geschmackseindrucks, oder zum Parfümieren eines Produkts, vorzugsweise eines erfindungsgemäßen Produkts.

Ein weiterer Aspekt der vorliegenden Erfindung betrifft ein Verfahren zum Herstellen eines erfindungsgemäßen Produkts, umfassend oder bestehend aus folgenden Schritten:
(a) Bereitstellen
   (a.1) eines erfindungsgemäßen Pulvers sowie
   (a.2) eines oder mehrerer weiterer Bestandteile, vorzugsweise eines oder mehrerer üblicher Bestandteile bzw. Inhaltsstoffe von Wasch- und Reinigungsmitteln, Körperpflegeprodukten, der Ernährung oder dem Genuss dienenden Zubereitungen, kosmetischen oder pharmazeutischen Zubereitungen oder parfümierten, parfümierenden oder zu parfümierenden Produkten, und
(b) Mischen der Komponenten (a.1) und (a.2).

Ein weiterer Aspekt der vorliegenden Erfindung betrifft ein Verfahren zum Parfümieren von Substraten, bevorzugt Haaren, Haut, Leder oder textilen Fasern, umfassend oder bestehend aus folgenden Schritten:
(a) Bereitstellen eines erfindungsgemäßen Pulvers oder Produkts wie hierin beschrieben, wobei mehrere oder sämtliche der Kapseln (K) einen oder mehrere Inhaltsstoffe aus der Gruppe bestehend aus Geruchsstoffen (vorzugsweise wie oben beschrieben) und Geschmacksstoffen enthalten, vorzugsweise in einer Menge, die ausreicht, um einen sensorischen, vorzugsweise einen Geruchs- und/oder Geschmackseindruck zu vermitteln, zu modifizieren oder zu verstärken und
(b) Auftragen des Pulvers bzw. Produkts auf die zu parfümierende(n) Substrate, bevorzugt Haare bzw. Haut bzw. Fasern bzw. Leder), vorzugsweise bezüglich der darin enthaltenen Kapseln (K) in einer sensorisch wirksamen Menge, vorzugsweise in einer Menge, die ausreicht, dass der Verbraucher eine oder mehrere Geruchs- oder Geschmacksnoten wahrnimmt.

Für bevorzugte Ausgestaltungen der oben beschriebenen Verwendungen und Verfahren gilt das oben im Zusammenhang mit den weiteren Aspekten der vorliegenden Erfindung Gesagte entsprechend, insbesondere das im Zusammenhang mit bevorzugten Ausgestaltungen Gesagte, ganz besonders betreffend die erfindungsgemäß (bevorzugt) zu verwendenden Kapseln, deren Hüllmaterialien und Inhaltsstoffe sowie die (bevorzugt) zu verwendenden kristallisierbaren Materialien.

Nachfolgend wird die Erfindung anhand von ausgewählten Beispielen näher erläutert. Sofern nicht anders angegeben, beziehen sich alle Angaben auf das Gewicht.

### Beispiel 1 (erfindungsgemäß):

450g Wasser werden auf ca. 33°C erwärmt. Dann werden 200g Natriumsulfat unter Rühren gelöst und langsam abgekühlt. Bei einsetzender Kristallisation werden dann 150g Kapseldispersion (710544 Symcap Tomcap, Symrise) zugesetzt und wird auf ca. 2°C abgekühlt und anschließend der Niederschlag abfiltriert. Der Filterkuchen wird dann getrocknet.

### Beispiel 2 (erfindungsgemäß):

450g Wasser werden auf ca. 40°C erwärmt, dann werden 167g Natriumcarbonat zugesetzt und unter Rühren gelöst. Anschließend werden 150g Kapseldispersion (710544 Symcap Tomcap, Symrise) und weitere 50g Wasser zugesetzt. Anschließend wird auf 1,0°C abgekühlt. Nach dem Abfiltrieren wird der Filterrückstand getrocknet.

### Beispiel 3 (erfindungsgemäß):

250g Wasser werden auf ca. 37°C erwärmt und anschließend 200g Natriumacetat unter Rühren gelöst. Anschließend wird die Mischung auf 26°C abkühlt und bei einsetzender Kristallisation wird 150 g Kapseldispersion (710544 Symcap Tomcap, Symrise) zugesetzt und auf 1,7°C in ca. 1h abgekühlt. Danach wird der Niederschlag abfiltriert und der Filterkuchen bei Raumtemperatur getrocknet.

### Beispiel 4 (Vergleichsbeispiel):

Eine Mischung aus 330g Wasser, 206g modifizierte Maisstärke (Capsul, Ingredion), 27,0g Maltose Monohydrat, 17,5g Luviskol K-30 und 150g Kapseldispersion (710544 Symcap Tomcap, Symrise) werden in einem Sprühtrockner (Büchi B 290) sprühgetrocknet (Inlet Temperatur 190°C, Outlet-Temperatur 90°C).

### Beispiel 5 (erfindungsgemäß):

450g Wasser werden auf ca. 80°C erwärmt. Dann werden 450g Harnstoff unter Rühren gelöst. Anschließend werden 150g Kapseldispersion (710544 Symcap Tomcap, Symrise) zugesetzt, anschließend wird auf Raumtemperatur abgekühlt und der Niederschlag abfiltriert. Der Filterkuchen wird anschließend getrocknet.

### Beispiel 6 (erfindungsgemäß):

500g Wasser werden auf ca. 40°C erwärmt. Dann werden 100 g Natriumsulfat unter Rühren gelöst. Anschließend werden 20 g Kapseldispersion (710544 Symcap Tomcap, Symrise) zugesetzt, anschließend wird auf 25 °C abgekühlt. Danach wird der Ansatz weiter auf ca. 1,7°C abgekühlt und dann weitere 20g Natriumsulfat als Impfkristall zugesetzt und der entstehende Niederschlag abfiltriert. Der Filterkuchen wird anschließend getrocknet.

### Beispiel 7: Vergleichsuntersuchungen

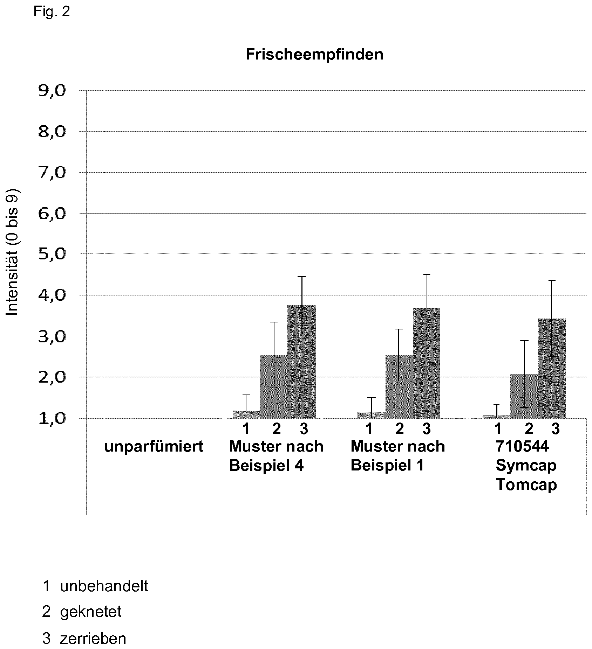

Zur Vorbereitung einer sensorischen Vergleichsuntersuchung wurden je 0,200g Muster nach Beispiel 4, 0,207g Muster nach Beispiel 1 und auf 40g Waschpulver gegeben und 0,08 g 710544 Symcap Tomcap (Symrise AG, Holzminden, Deutschland) auf je 40g Waschpulver (Denk mit Vollwaschmittel Ultra Sensitive Pulver, dm-drogerie markt GmbH + Co. KG, Karlsruhe, Deutschland) und gemischt. Anschließend wurden die so hergestellten Muster auf jeweils 2 kg Frotteelappen (Fa. Karl Heinz Hesse GmbH, Dransfeld, Germany, Waschlappen TB1, 80% BW, 20% PES, 30x30 cm) in einer handelsüblichen europäischen Waschmaschine bei 40°C verwaschen. Anschließend wurden die Frotteelappen bei Raumtemperatur auf der Leine getrocknet. Die anonymisierten Proben wurden dann von einem 14-köpfigen Expertenpanel jeweils ohne mechanische Belastung (unbehandelt), nach dem leichten Kneten der Wäsche (geknetet) und nach dem Reiben der Lappen gegeneinander (zerrieben) abgerochen und die Duftintensität anhand einer Skala von 1-9 bewertet. Das Ergebnis (vgl. Fig. 2) zeigt, dass die Freisetzung der Kapseln bzw. von deren Inhaltsstoffen im Waschpulver, hergestellt durch das erfindungsgemäße Verfahren, im Vergleich mit sprühgetrockneten Kapseln (gemäß Beispiel 4) oder im Vergleich mit der Verwendung von flüssiger Kapseldispersion allein (710544 Symcap Tomcap) nicht vermindert wird.

### Beispiel 8 (erfindungsgemäß):

500 g Wasser werden auf ca. 33°C erwärmt. Dann werden 200 g Natriumsulfat unter Rühren gelöst. Anschließend wird die Mischung langsam abgekühlt die Kristallisation beginnt und sich erste kleine Kristalle bilden. Dann wird 198 g Folco Microdeur (Follmann GmbH & Co. KG, Minden, Germany) zugesetzt und auf 1,7 °C abgekühlt. Anschließend wird der Niederschlag abfiltriert und der Filterkuchen getrocknet.

## Patentansprüche

1. Verfahren zur Herstellung eines Pulvers bestehend aus oder umfassend eine Vielzahl von in Kristallen eingeschlossenen, einen oder mehrere Inhaltsstoffe enthaltenden Kapseln (K), bestehend aus oder umfassend folgende Schritte:
(i) Bereitstellen von einem oder mehreren Lösemitteln, Bereitstellen von einem oder mehreren kristallisierbaren Materialien und Bereitstellen von einem oder mehreren einen oder mehrere Inhaltsstoffe enthaltenden Kapseln (K),
(ii) optional: Temperaturerhöhung des/der Lösemittel(s),
(iii) Hinzufügen
(iii.1) des/der bereitgestellten kristallisierbaren Materials/Materialien und
(iii.2) optional: der bereitgestellten Kapseln (K), vor, nach oder gleichzeitig mit dem/den kristallisierbaren Materialien,
zu dem/den Lösemittel(n) und Lösen des/der bereitgestellten kristallisierbaren Materials/Materialien in dem/den Lösemittel(n), um eine Mischung (M1) zu erhalten,
(iv) Reduzieren der Temperatur der Mischung (M1) auf eine Temperatur unterhalb der oder bis zur Kristallisation des/der bereitgestellten kristallisierbaren Materials/Materialien,
(v) Hinzufügen der bereitgestellten Kapseln (K) bzw. weiterer Kapseln (K), wobei Schritt (v) im Falle der Durchführung des Schrittes (iii.2) optional ist,
(vi) weiteres Reduzieren der Temperatur der Mischung (M1), um eine Mischung (M2) zu erhalten, die eine Vielzahl von in Kristallen eingeschlossenen, einen oder mehrere Inhaltsstoffe enthaltenden Kapseln (K), umfasst,
(vii) Sammeln von Kristallen enthaltend, einen oder mehrere Inhaltsstoffe enthaltenden Kapseln (K).

2. Verfahren nach Anspruch 1, wobei das/ein, mehrere oder sämtliche Lösungsmittel ausgewählt ist/sind aus der Gruppe bestehend aus Wasser und mit Wasser mischbaren Lösungsmitteln, bevorzugt Ethanol, Isopropanol, Aceton, DMSO.

3. Verfahren nach Anspruch 1 oder 2, wobei sich das/ein, mehrere oder sämtliche kristallisierbare Material(ien) in einem mit Wasser mischbaren Lösungsmittel oder mit Wasser kristallisieren lässt/lassen und/oder ausgewählt ist/sind aus der Gruppe bestehend aus Salzen, bevorzugt Alkali- und Erdalkalimetallsalze, besonders bevorzugt Natriumsalze, insbesondere bevorzugt Natriumsulfat, Natriumchlorid oder Natriumcarbonat, Zuckern, Harnstoff.

4. Verfahren nach einem der vorangehenden Ansprüche, wobei mehrere oder sämtliche der Kapseln (K) einen oder mehrere Inhaltsstoffe aus der Gruppe bestehend aus Geruchsstoffen, Geschmacksstoffen, kosmetischen Inhaltsstoffen, pharmazeutischen Wirkstoffen, Insektenrepellentien, Inhaltsstoffen für Mund- und Zahnpflegemittel, Latentwärmespeichern und Adsorbentien, enthalten.

5. Verfahren nach einem der vorangehenden Ansprüche, wobei mehrere oder sämtliche der Kapseln (K) ein Wandmaterial aufweisen, das aus einem oder mehreren der Substanzen ausgewählt aus der Gruppe bestehend aus natürlichen, halbsynthetischen und vollsynthetischen Hüllmaterialien, bevorzugt aus Melamin Formaldehyd Harzen, Gelatine, Alginaten, Polyurethanen, Polyamiden und Polyharnstoffen besteht oder eine oder mehrere solche Substanzen umfasst oder auf einer oder mehreren solchen Substanzen basiert.

6. Verfahren nach einem der vorangehenden Ansprüche, wobei in Schritt (ii), sofern vorhanden, das/die Lösemittel auf eine Temperatur im Bereich von 23 bis 50 °C, vorzugsweise im Bereich von 25 bis 40 °C, besonders bevorzugt im Bereich von 30 bis 35 °C, erhöht wird/werden.

7. Verfahren nach einem der vorangehenden Ansprüche, wobei Schritt (iii) ein Rühren umfasst, um das/die kristallisierbare(n) Material(ien) in dem/den Lösemitteln zu lösen oder dies zu erleichtern, vorzugsweise mit einer Rührgeschwindigkeit im Bereich von 100 bis 1.871 U/Min., bevorzugt im Bereich von 500 bis 1300 U/Min., besonders bevorzugt im Bereich von 1000 bis 1200 U/Min., vorzugsweise mittels eines Leitstrahlrührers.

8. Verfahren nach einem der vorangehenden Ansprüche, wobei in Schritt (vi) die Temperatur der Mischung (M1) auf eine Temperatur von < 20 °C, vorzugsweise < 15 °C, weiter vorzugsweise < 10 °C, besonders bevorzugt < 5 °C, reduziert wird, wobei das Reduzieren der Temperatur vorzugsweise über einen Zeitraum im Bereich von 0,25 bis 3 Stunden, bevorzugt 0,5 bis 2 Stunden, besonders bevorzugt über einen Zeitraum von 0,75 bis 1,5 Stunden, erfolgt.

9. Verfahren nach einem der vorangehenden Ansprüche, wobei Schritt (vii) einen, zwei oder sämtliche der folgenden Schritte umfasst oder daraus besteht:
- Dekantieren des/der Lösemittel(s),
- Abtrennen der Kristalle,
- Trocknen der in Schritt (vi) erhaltenen Mischung (M2) und/oder der abfiltrierten Kristalle,
um ein Pulver bestehend aus oder umfassend eine Vielzahl von in Kristallen eingeschlossenen, einen oder mehrere Inhaltsstoffe enthaltenden Kapseln (K) zu erhalten.

10. Verfahren nach einem der vorangehenden Ansprüche, wobei in Schritt (i) das Gewichtsverhältnis der Gesamtmenge an Lösemittel(n) zur Gesamtmenge an kristallisierbarem/n Material(ien) zur Gesamtmenge an Kapseln (K) und/oder an Kapseldispersion, falls die Kapseln in Form einer solchen bereitgestellt und hinzugefügt werden, in folgendem Bereich liegt: 1-10 : 0.5-5 : 0.1-3, vorzugsweise in folgendem Bereich: 3-7 : 1-3 : 0.5-2.

11. Pulver bestehend aus oder umfassend eine Vielzahl von in Kristallen eingeschlossenen, einen oder mehrere Inhaltsstoffe enthaltenden Kapseln, hergestellt oder herstellbar durch ein Verfahren nach einem oder mehreren der vorangehenden Ansprüche.

12. Produkt ausgewählt aus der Gruppe bestehend aus Wasch- und Reinigungsmitteln, Körperpflegeprodukten, der Ernährung oder dem Genuss dienenden Zubereitungen, kosmetischen oder pharmazeutischen Zubereitungen, parfümierten, parfümierenden oder zu parfümierenden Produkten, vorzugsweise Duftspülern, wobei das Produkt ein Pulver nach Anspruch 11 umfasst.

13. Verwendung eines Pulvers nach Anspruch 11, wobei mehrere oder sämtliche der Kapseln (K) einen oder mehrere Inhaltsstoffe aus der Gruppe bestehend aus Kühlwirkstoffen, Geruchsstoffen und Geschmacksstoffen enthalten, vorzugsweise in einer Menge, die ausreicht, um eine Kühlwirkung hervorzurufen und/oder um einen sensorischen, vorzugsweise einen Geruchs- und/oder Geschmackseindruck zu vermitteln, zu modifizieren oder zu verstärken, zum Vermitteln, Modifizieren oder Verstärken eines sensorischen, vorzugsweise eines Geruchs- und/oder Geschmackseindrucks, oder zum Parfümieren eines Produkts, vorzugsweise eines Produkts nach Anspruch 12.

14. Verfahren zum Herstellen eines Produkts nach Anspruch 12, umfassend oder bestehend aus folgenden Schritten:
(a) Bereitstellen
(a.1) eines Pulvers nach Anspruch 11 sowie
(a.2) eines oder mehrerer weiterer Bestandteile, und
(b) Mischen der Komponenten (a.1) und (a.2).

15. Verfahren zum Parfümieren von Substraten, bevorzugt Haaren, Haut, Leder oder textilen Fasern, umfassend oder bestehend aus folgenden Schritten:
(a) Bereitstellen eines Pulvers nach Anspruch 11 oder eines Produkts nach Anspruch 12, wobei mehrere oder sämtliche der Kapseln (K) einen oder mehrere Inhaltsstoffe aus der Gruppe bestehend aus Geruchsstoffen und Geschmacksstoffen enthalten, vorzugsweise in einer Menge, die ausreicht, um einen sensorischen, vorzugsweise einen Geruchs- und/oder Geschmackseindruck zu vermitteln, zu modifizieren oder zu verstärken und
(b) Auftragen des Pulvers bzw. Produkts auf die zu parfümierende(n) Substrate, bevorzugt Haare bzw. Haut bzw. Fasern bzw. Leder), vorzugsweise bezüglich der darin enthaltenen Kapseln (K) in einer sensorisch wirksamen Menge, vorzugsweise in einer Menge, die ausreicht, dass der Verbraucher eine oder mehrere Geruchs- oder Geschmacksnoten wahrnimmt.

## Claims

1. Method for producing a powder consisting of or comprising a plurality of capsules (K), which are enclosed in crystals and contain one or more ingredient(s), consisting of or comprising the following steps:
(i) providing one or more solvent(s),
providing one or more crystallisable material(s) and
providing one or more capsule(s) (K) containing one or more ingredient(s),
(ii) optionally: increasing the temperature of the solvent(s),
(iii) adding
(iii.1) the provided crystallisable material(s) and
(iii.2) optionally: the provided capsules (K) before, after or simultaneously with the crystallisable material(s)
to the solvent(s) and dissolving the provided crystallisable material(s) in the solvent(s) to obtain a mixture (M1),
(iv) reducing the temperature of mixture (M1) to a temperature below or until the crystallization of the provided crystallisable material(s),
(v) adding the provided capsules (K) or, respectively, further capsules (K), wherein step (v) is optional in case step (iii.2) is performed,
(vi) further reducing the temperature of mixture (M1) to obtain a mixture (M2) comprising a plurality of capsules (K), which are enclosed in crystals and contain one or more ingredient(s),
(vii) collecting crystals comprising capsules (K), which contain one or more ingredient(s).

2. Method according to claim 1, wherein the, one, more or all solvent(s) is / are selected from the group consisting of water and solvents, which are mixable with water, preferably ethanol, isopropanol, acetone, DMSO.

3. Method according to claim 1 or 2, wherein the, one, more or all crystallisable material(s) is / are crystallisable in a solvent, which is mixable with water, or in water and / or is / are selected from the group consisting of salts, preferably alkaline and alkaline earth metal salts, particularly preferably sodium salts, particularly preferably sodium sulphate, sodium chloride or sodium carbonate, sugars, urea.

4. Method according to one of the previous claims, wherein several or all of the capsules (K) contain one or more ingredient(s) selected from the group consisting of odorants, flavours, cosmetic ingredients, pharmaceutically active substances, insect repellents, ingredients for oral and dental care products, latent heat accumulator and adsorbents.

5. Method according to one of the previous claims, wherein several or all of the capsules (K) contain a wall material, consisting of one or more of the substances from the group consisting of natural, semi-synthetic and fully synthetic coating materials, preferably of melamin formaldehyde resins, gelatin, alginates, polyurethanes, polyamides and polyureas, or comprising one or more of such substances or based on one or more of such substances.

6. Method according to one of the previous claims, wherein in step (ii), if present, the solvent(s) is / are heated to a temperature in the range of from 23 to 50 °C, preferably in the range of from 25 to 40 °C, particularly preferably in the range of from 30 to 35 °C.

7. Method according to one of the previous claims, wherein step (iii) comprises a stirring to dissolve the crystallisable material(s) in the solvent(s) or to facilitate the same, preferably with a stirring speed in the range of from 100 to 1,871 rpm [U/min], preferably in the range of from 500 to 1300 rpm [U/min], particularly preferably in the range of from 1000 to 1200 rpm [U/min], preferably with a jet stream mixer.

8. Method according to one of the previous claims, wherein in step (vi) the temperature of the mixture (M1) is reduced to a temperature of < 20 °C, preferably < 15 °C, further preferably < 10 °C, particularly preferably < 5 °C,
wherein the reducing of the temperature is preferably performed over a time in the range of from 0.25 to 3 hours, preferably 0.5 to 2 hours, particularly preferably over a time of from 0.75 to 1.5 hours.

9. Method according to one of the previous claims, wherein step (vii) comprises or consists of one, two or all of the following steps:
- decanting the solvent(s),
- separating the crystals,
- drying the mixture (M2) obtained in step (vi) and / or the filtered crystals,
to obtain a powder consisting of or comprising a plurality of capsules (K), which are enclosed in crystals and contain one or more ingredient(s).

10. Method according to one of the previous claims, wherein in step (i) the weight ratio of the total amount of solvent(s) to the total amount of crystallisable material(s) to the total amount of capsules (K) and / or capsule dispersion, in case the capsules are provided and added in form of a capsule dispersion, is in the following range: 1-10 : 0.5-5 : 0.1-3, preferably in the following range: 3-7 : 1-3 : 0.5-2.

11. Powder consisting of or comprising a plurality of capsules (K), which are enclosed in crystals and contain one or more ingredient(s), produced or producible by a method according to one or more of the previous claims.

12. Product selected from the group consisting of washing and cleaning agents, body care products, compositions for nutrition or pleasure, cosmetic or pharmaceutical compositions, perfumed or perfuming products, products to be perfumed, preferably rim blocks, wherein the product comprises a powder according to claim 11.

13. Use of a powder according to claim 11, wherein several or all of the capsules (K) contain one or more ingredient(s) of the group consisting of cooling agents, odorants and flavours, preferably in an amount sufficient for causing a cooling effect and / or for imparting, modifying or improving a sensory, preferably an olfactory and / or gustatory impression, for imparting, modifying or increasing a sensory, preferably an olfactory and / or gustatory impression, or for perfuming a product, preferably a product according to claim 12.

14. Method for producing a product according to claim 12, comprising of or consisting the following steps:
(a) providing
(a.1) a powder according to claim 11, as well as
(a.2) one or more further components, and
(b) mixing components (a.1) and (a.2).

15. Method for perfuming substrates, preferably hair, skin, leather or textile fibres, comprising or consisting of the following steps:
(a) providing a powder according to claim 11 or a product according to claim 12, wherein several or all of the capsules (K) contain one or more ingredient(s) of the group consisting of odorants and flavours, preferably in an amount sufficient for imparting, modifying or increasing a sensory, preferably an olfactory and / or gustatory impression and
(b) applying the powder or, respectively, product to the substrate(s) to be perfumed, preferably hair or, respectively, skin or, respectively, fibres or, respectively, leather, preferably in a sensorially effective amount with regard to the capsules (K) contained in the powder or, respectively, product, preferably in an amount sufficient for a consumer to perceive one or more olfactory or gustatory scent(s).

## Revendications

1. Procédé de production d'une poudre consistant en ou comprenant une pluralité de capsules (K) enfermées dans des cristaux et contenant un ou plusieurs ingrédient(s), consistant en ou comprenant les étapes suivantes:
(i) fournir un ou plusieurs solvant(s),
fournir un ou plusieurs matériau(x) cristallisable(s) et
fournir une ou plusieurs capsule(s) (K) contenant un ou plusieurs ingrédients,
(ii) en option: augmenter la température du ou des solvant(s),
(iii) ajouter
(iii.1) le ou les matériau(x) cristallisable(s) fourni(s) et
(iii.2) en option: les capsules (K) fournies, avant, après ou simultanément avec le ou les matériaux cristallisables,
au(x) solvant(s) et dissoudre le ou les matériau(x) cristallisable(s) fourni(s) dans le(s) solvant(s) pour obtenir un mélange (M1),
(iv) réduire la température du mélange (M1) à une température inférieure ou jusqu'à la cristallisation du ou des matériau(x) cristallisable(s) fourni(s),
(v) ajouter les capsules (K) fournies ou bien d'autres capsules (K), l'étape (v) étant optionnelle dans le cas de la mise en œuvre de l'étape (iii.2),
(vi) réduire encore la température du mélange (M1) afin d'obtenir un mélange (M2) qui comprend une pluralité de capsules (K) enfermées dans des cristaux et contenant un ou plusieurs ingrédients,
(vii) collecter des cristaux contenant des capsules (K) contenant un ou plusieurs ingrédients.

2. Procédé selon la revendication 1, dans lequel le/un, plusieurs ou tous les solvant(s) est/sont choisi(s) dans le groupe constitué par l'eau et les solvants miscibles à l'eau, de préférence l'éthanol, l'isopropanol, l'acétone, le DMSO.

3. Procédé selon la revendication 1 ou 2, dans lequel le/un matériau cristallisable, plusieurs ou tous les matériaux cristallisables peut/peuvent être cristallisé(s) dans un solvant miscible à l'eau ou avec de l'eau et/ou est/sont choisi(s) dans le groupe constitué par les sels, de préférence les sels de métaux alcalins et alcalino-terreux, de manière particulièrement préférée les sels de sodium, de manière particulièrement préférée le sulfate de sodium, le chlorure de sodium ou le carbonate de sodium, les sucres, l'urée.

4. Procédé selon l'une quelconque des revendications précédentes, dans lequel plusieurs ou toutes les capsules (K) contiennent un ou plusieurs ingrédients du groupe constitué par les substances odorantes, les agents de sapidité, les ingrédients cosmétiques, les substances actives pharmaceutiques, les insectifuges, les ingrédients pour les produits de soins bucco-dentaires, les accumulateurs de chaleur latente et les adsorbants.

5. Procédé selon l'une quelconque des revendications précédentes, dans lequel plusieurs ou l'ensemble des capsules (K) présentent un matériau de paroi qui est composé d'une ou de plusieurs des substances choisies dans le groupe constitué de matériaux d'enveloppe naturels, semi-synthétiques et entièrement synthétiques, de préférence de résines mélamine-formaldéhyde, de gélatine, d'alginates, de polyuré-thanes, de polyamides et de polyurées, ou comprend une ou plusieurs de ces substances ou est à base d'une ou de plusieurs de ces substances.

6. Procédé selon l'une quelconque des revendications précédentes, dans lequel, à l'étape (ii), le(s) solvant(s) - s'il(s) est (sont) présent(s), est (sont) porté(s) à une température comprise entre 23 et 50 °C, de préférence comprise entre 25 et 40 °C, de manière particulièrement préférée comprise entre 30 et 35 °C.

7. Procédé selon l'une quelconque des revendications précédentes, dans lequel l'étape (iii) comprend une agitation afin de dissoudre le ou les matériau(x) cristallisable(s) dans le(s) solvant(s) ou pour faciliter ceci, de préférence à une vitesse d'agitation comprise entre 100 et 1.871 tours/min, de préférence comprise entre 500 et 1300 tours/min, de manière particulièrement préférée comprise entre 1000 et 1200 tours/min, de préférence au moyen d'un agitateur à jet dirigé.

8. Procédé selon l'une quelconque des revendications précédentes, dans lequel, à l'étape (vi), la température du mélange (M1) est réduite à une température < 20 °C, de préférence < 15 °C, en outre de préférence < 10 °C, de manière particulièrement préférée < 5 °C, dans lequel la réduction de la température est réalisée de préférence sur une durée comprise entre 0,25 et 3 heures, de préférence entre 0,5 et 2 heures, de manière particulièrement préférée sur une durée comprise entre 0,75 et 1,5 heures.

9. Procédé selon l'une quelconque des revendications précédentes, dans lequel l'étape (vii) comprend ou consiste en une, deux ou toutes les étapes suivantes:
- décanter le(s) solvant(s),
- séparer les cristaux,
- sécher le mélange (M2) obtenu à l'étape (vi) et/ou les cristaux filtrés,
afin d'obtenir une poudre consistant en ou comprenant une pluralité de capsules (K) enfermées dans des cristaux et contenant un ou plusieurs ingrédient(s).

10. Procédé selon l'une quelconque des revendications précédentes, dans lequel, à l'étape (i), le rapport pondéral de la quantité totale de solvant(s) à la quantité totale de matériau(x) cristallisable(s) à la quantité totale de capsules (K) et/ou de dispersion de capsule, si les capsules sont fournies et ajoutées sous la forme d'une telle dispersion, se situe dans la plage suivante: 1 - 10 : 0,5 - 5 : 0,1 - 3, de préférence dans la plage suivante: 3 - 7 : 1 - 3 : 0,5 - 2.

11. Poudre constituée de ou comprenant une pluralité de capsules enfermées dans des cristaux et contenant un ou plusieurs ingrédients, produite ou apte à être produite par un procédé selon une ou plusieurs des revendications précédentes.

12. Produit choisi dans le groupe constitué par les agents de lavage et de nettoyage, les produits de soins corporels, les préparations servant à la nutrition ou au plaisir, les préparations cosmétiques ou pharmaceutiques, les produits parfumés, parfumants ou à parfumer, de préférence les rinceurs parfumés, dans lequel le produit comprend une poudre selon la revendication 11.

13. Utilisation d'une poudre selon la revendication 11, dans laquelle plusieurs ou l'ensemble des capsules (K) contiennent un ou plusieurs ingrédients du groupe constitué par les substances actives de refroidissement, les substances odorantes et les agents de sapidité, de préférence en une quantité qui est suffisante pour provoquer un effet de refroidissement et/ou pour conférer, modifier ou intensifier une impression sensorielle, de préférence une impression olfactive et/ou gustative, afin de conférer, modifier ou intensifier une impression sensorielle, de préférence une impression olfactive et/ou gustative ou afin de parfumer un produit, de préférence un produit selon la revendication 12.

14. Procédé de production d'un produit selon la revendication 12, comprenant ou consistant en les étapes suivantes:
(a) fournir
(a.1) une poudre selon la revendication 11, ainsi que
(a.2) un ou plusieurs autres composants, et
(b) mélanger les composants (a.1) et (a.2).

15. Procédé destiné à parfumer des substrats, de préférence les cheveux, la peau, le cuir ou des fibres textiles, comprenant ou consistant en les étapes suivantes:
(a) fournir une poudre selon la revendication 11 ou un produit selon la revendication 12, dans lequel plusieurs ou l'ensemble des capsules (K) contiennent un ou plusieurs ingrédients du groupe constitué de substances odorantes et d'agents de sapidité de préférence en une quantité qui est suffisante pour conférer, modifier ou intensifier une impression sensorielle, de préférence une impression olfactive et/ou gustative, et
(b) appliquer la poudre ou bien le produit sur le(s) substrat(s) à parfumer, de préférence les cheveux ou bien la peau ou bien les fibres ou bien le cuir, de préférence en ce qui concerne les capsules (K) qui y sont contenues en une quantité sensoriellement efficace, de préférence en une quantité qui est suffisante pour que le consommateur perçoive une ou plusieurs notes odorantes ou gustatives.
